# EUROPEAN PATENT APPLICATION

(11) **EP 2 057 989 A1**
(43) Date of publication of application: **13.05.2009**
(21) Application number: 07793042.8
(22) Date of filing: 28.08.2007
(51) Int. Cl.: A61K 31/407, A61K 9/19, A61P 31/04, C07D 477/00

(54) **FREEZE-DRIED PREPARATION OF 1-METHYLCARBAPENEM**

(30) Priority: 29.08.2006 JP 2006231438
(71) Applicant: Daiichi Sankyo Company, Limited, Chuo-ku Tokyo 103-0023 (JP)
(72) Inventor: KIKUCHI, Takayuki, Shinomiya, Hiratsuka-shi, Kanagawa 254-0014 (JP); KIHARA, Fumihiro, Shinomiya, Hiratsuka-shi, Kanagawa 254-0014 (JP); SUZUKI, Masahiko, Shinomiya, Hiratsuka-shi, Kanagawa 254-0014 (JP)
(74) Representative: Smyth, Gyles Darren
(86) International application number: PCT/JP2007/066590
(87) International publication number: WO 2008/026556

(57) **Abstract**

An object of the present invention is to produce a lyophilized preparation comprising (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid or a pharmacologically acceptable salt thereof as a carbapenem compound which has a 1-alkylpyrrolidine structure and possesses a superior antimicrobial activity, and sodium chloride.

## Description

### [TECHNICAL FIELD]

The present invention relates to a lyophilized preparation comprising a carbapenem compound, which has a 1-alkylpyrrolidine structure and possesses a superior antimicrobial activity, and sodium chloride, and to a method of production thereof.

### [BACKGROUND ART]

In the field of pharmaceutical preparations, methods of producing a preparation by lyophilization are widely used. However, since carbapenem compounds are substances that are chemically decomposed easily during storage, it is often required to take measures in order to provide a preparation with sufficient stability.

Non-Patent Document 1 discloses a lyophilized preparation which formulates panipenem, which is a carbapenem antibiotic having a 1-acetoimidoylpyrrolidine structure represented by the following formula: 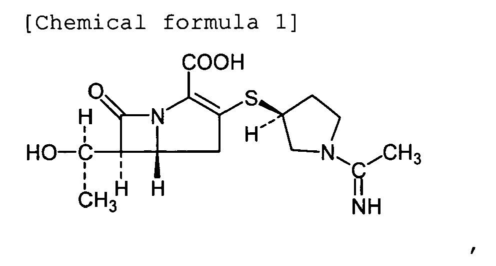 and betamipron, which is an organic anion transport inhibitor, in 1:1 (weight ratio). Further, the document also discloses that formulation with sodium chloride improves the stability of panipenem in the lyophilized preparation.

On the other hand, Patent Document 1 discloses a lyophilized preparation which blends a 1-methylcarbapenem antibiotic represented by the following formula: 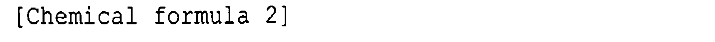 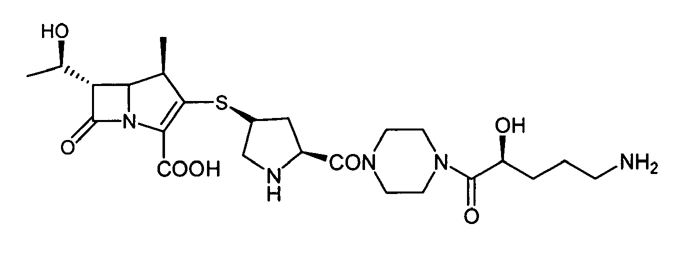 with inorganic salts (sodium chloride), and in Table 1 of the Example, it is disclosed that in the case where the formulation is not performed with saccharides, formulation with sodium chloride decreases the stability of the lyophilized preparation.

Accordingly, whether formulation with inorganic salts such as sodium chloride improves or decreases the stability of the lyophilized preparation, or has almost no effect on the stability, would differ according to the type of the carbapenem compound which is the active ingredient and other production conditions. Therefore it cannot be predicted by a person skilled in the art, but becomes understood only after conducting tests.
[Non-Patent Document 1] Antibiotics & Chemotherapy Vol. 10, No. 7, (1333-1341) 89-97, 1994
[Patent Document 1] Japanese Patent Application (Kokai) No. Hei 8-231398

### [DISCLOSURE OF THE INVENTION]

### [PROBLEMS TO BE SOLVED BY THE INVENTION]

As a result of conducting various studies to solve the problem of providing a stable lyophilized preparation comprising a particular carbapenem compound, which has a particular 1-alkylpyrrolidine structure, or a salt thereof, as an active ingredient, the inventors of the present invention found that stability is improved by allowing inclusion of sodium chloride, and that the carbapenem compound or the salt thereof can be improved in long-term storage stability, thereby leading to completion of the present invention.

### [MEANS FOR SOLVING THE PROBLEMS]

The present invention is a lyophilized preparation comprising (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (hereinafter referred to as compound (I)) or a pharmacologically acceptable salt thereof as a carbapenem compound, and sodium chloride.

In addition, the present invention is a method of production of a lyophilized preparation, comprising lyophilization of an aqueous solution of compound (I) or a pharmacologically acceptable salt thereof in the presence of sodium chloride.

Compound (I) of the present invention can be converted into a pharmacologically acceptable salt if necessary.

In a case where carbapenem compound (I) can form a salt with an acidic compound, such an acidic compound can be, for example, an inorganic acid such as hydrofluoric acid, hydrochloric acid, hydrobromic acid, hydroiodic acid, sulfuric acid, nitric acid, phosphoric acid or carbonic acid; an organic carboxylic acid such as formic acid, acetic acid, trifluoroacetic acid, oxalic acid or phthalic acid; or an organic sulfonic acid such as methanesulfonic acid, trifluoromethanesulfonic acid, benzenesulfonic acid or p-toluenesulfonic acid; and is preferably an inorganic acid, and more preferably hydrochloric acid, sulfuric acid or carbonic acid.

Further, compound (I) of the present invention has a carboxyl group, and thus can form a salt with a basic substance. Such a salt can be, for example, an alkali metal salt such as sodium salt, potassium salt or lithium salt; an alkali earth metal salt such as calcium salt or magnesium salt; or ammonium salt, and is preferably lithium salt, sodium salt, potassium salt or magnesium salt.

Here, when compound (I) of the present invention is exposed to the atmosphere, lyophilized from aqueous solution or recrystallized, it may absorb moisture, addition of adsorbed water may occur, and hydrates may be generated. Such hydrates are also included in the present invention.

In addition, there are cases where compound (I) of the present invention absorbs a particular type of solvent and forms solvates, and such solvates are also included in the present invention.

Compound (I) of the present invention is a compound disclosed in Japanese Patent Application (Kokai) No. Hei 10-204086 and Japanese Patent Application (Kokai) No. Hei 11-071277, and possesses a strong antimicrobial activity with respect to a wide range of bacteria including gram-positive bacteria and gram-negative bacteria.

The lower the pH (the stronger the acidity), the higher the solubility of compound (I) of the present invention in water. On the other hand, the solubility becomes low as the aqueous solution becomes close to neutral. The method for measuring the solubility of compound (I) is as described below. Compound (I) possessed solubility sufficient for the production of the preparation at pH 7 or lower, and the solubility increased in accordance with the decrease in pH.

### Method for measuring solubility of compound (I):

Compound (I) (in an amount so that concentration of compound (I) in the aqueous solution becomes 300 mg/g) is weighed in a beaker. 15mL of water for injection cooled to 5°C is added, and the solution is stirred using a stirrer under ice-cold conditions for 30 minutes. While stirring, adjustment to the predetermined pH is conducted by dropwise addition of 1N hydrochloric acid or 1N sodium hydroxide aqueous solution, and the pH is maintained. After stirring is completed, a filtration filter (for example, Ekicrodisc 0.45µm HT-Tuffryn, Gelman Science Japan, Ltd.) is used for filtration, and the amount of compound (I) in the filtrate is measured by the quantitative HPLC method which is similar to Test Example 1 described later, and thus solubility of compound (I) is obtained.

In the present invention, the amount of sodium chloride added is not particularly limited, and the lower limit may be 0.1 equivalent (mol), preferably 0.5 equivalent (mol), and more preferably 0.7 equivalent (mol) with respect to compound (I), and the upper limit may be 2.5 equivalent (mol), preferably 2.0 equivalent (mol), and more preferably 1.8 equivalent (mol) with respect to compound (I). Here, when the amount of sodium chloride added is too large, there is a fear that degradation in the quality of the lyophilized preparation (poor appearance) such as occurrence of cracks in the lyophilized product (lyophilized cake) may arise.

### [EFFECT OF THE INVENTION]

The lyophilized preparation of the present invention has high storage stability compared with a lyophilized preparation which does not contain sodium chloride, and, even upon long-term storage, the proportion of the active ingredient which remains is high. Further, it is useful since it rapidly dissolves by addition of injection solution at the time of usage.

### [BEST MODE FOR CARRYING OUT THE INVENTION]

Compound (I), which is the active ingredient of the present invention, can be produced in accordance with the methods disclosed in Japanese Patent Application (Kokai) No. Hei 10-204086, Japanese Patent Application (Kokai) No. Hei 11-071277, Japanese Patent Application (Kokai) No. Hei 11-315021 and Japanese Patent Application (Kokai) No. 2002-212183, or methods based thereon.

The lyophilized preparation of the present invention can be produced by preparing an aqueous solution (bulk solution) by dissolving compound (I) or a pharmacologically acceptable salt thereof and sodium chloride in an appropriate aqueous solvent such as water for injection, and then lyophilizing this bulk solution by conventional methods. In particular, the lyophilized preparation of the present invention can be produced by preparing an aqueous solution (bulk solution) by dissolving compound (I), sodium chloride and other additive(s) if necessary in an appropriate aqueous solvent such as water for injection, then aseptically filtering this bulk solution and filling the filtrate into containers such as vials or ampules, followed by lyophilization. In addition, the preparation can also be produced by aseptically filtering the bulk solution followed by lyophilization of the filtrate as it is to obtain a lyophilized powder, and then filling the powder into containers such as vials or ampules. Alternatively, the powder can be filled as a kit formulation.

In the aforementioned methods of production, the solubility of compound (I) should be taken into consideration, and it is preferable to adjust the pH of the bulk solution by using the pH regulators described later, so that the pH satisfies the lower limit of pH 5.5, preferably 6.0, more preferably 6.1, and the upper limit of pH 7.0, preferably 6.6, more preferably 6.5.

In addition, additives for preparations that are available to a person skilled in the art, such as excipients, stabilizers, antioxidants, dissolution adjuvants, buffers, pH regulators, isotonization agents, and/or dissolution agents, can be added if necessary, followed by lyophilization.

Excipients of the present invention can be, for example, saccharides such as purified sucrose, lactose, maltose, trehalose and mannitol; dextran; pullulan; phosphate derivatives such as calcium phosphate; carbonate derivatives such as calcium carbonate; or sulfate derivatives such as calcium sulfate, preferably saccharides, and more preferably lactose.

Stabilizers of the present invention can be, for example, saccharides such as glucose or purified sucrose; edetic acids such as sodium edetate or tetrasodium edetate; paraoxybenzoic acid esters such as methylparaben or propylparaben; alcohols such as chlorobutanol, benzyl alcohol or phenylethyl alcohol; benzalkonium chloride; phenols such as phenol or cresol; thimerosal; acetic anhydride; or sorbic acid, preferably edetic acids, and more preferably sodium edetate.

Antioxidants of the present invention can be, for example, vitamins such as L-ascorbic acid, tocopherol acetate or vitamin E; sodium nitrite; sodium bisulfite; sodium sulfate; edetic acids such as sodium edetate or tetrasodium edetate, preferably edetic acids, and more preferably sodium edetate.

Dissolution adjuvants of the present invention can be, for example, nonionic surfactants such as propylene glycol, polyoxyethylene-hardened castor oil or polysorbate 80, preferably polysorbate 80.

Buffers of the present invention can be, for example, acetic acid; phosphates such as phosphoric acid, sodium hydrogenphosphate or potassium dihydrogenphosphate; or citric acid, preferably phosphoric acids, and more preferably sodium hydrogenphosphate.

pH regulators of the present invention can be, for example, citric acid; acetic acid; sodium hydroxide; hydrochloric acid; sodium bicarbonate; phosphates such as phosphoric acid, sodium hydrogenphosphate or potassium dihydrogenphosphate, preferably sodium hydroxide or hydrochloric acid, more preferably hydrochloric acid.

Isotonization agents of the present invention can be, for example, salts such as sodium chloride or potassium chloride; saccharides such as glucose or sucrose; or glycerin, preferably saccharides, and more preferably sucrose.

Dissolution agents of the present invention can be, for example, dissolution liquids for injection such as water for injection, physiological saline solution or glucose solution, preferably water for injection.

The lyophilized preparation can be produced, for example, in accordance with the following procedure. Production of the lyophilized preparation of the present invention is not limited to these procedures.

### (1) Bulk solution (when total amount is 100 mL)

Compound (I) and sodium chloride are weighed in a container. The container is ice-cooled, followed by addition of 30 mL of water for injection which was cooled to 5°C beforehand. Compound (I) is dispersed and sodium chloride is dissolved by stirring. Compound (I) is completely dissolved by dropwise addition of hydrochloric acid which was cooled beforehand to the mixture while stirring under ice-cold conditions. Further, pH is adjusted by using hydrochloric acid which was cooled beforehand and, if necessary, an aqueous sodium hydroxide solution which was cooled beforehand. Water for injection which was cooled beforehand is added to the solution under ice-cooled conditions so that the total amount becomes 100 mL. The solution obtained is filtered aseptically to give the bulk solution.

In the aforementioned procedures, the concentration of hydrochloric acid is usually 0.4 to 20N, preferably 0.75 to 5N.

In the aforementioned procedures, the concentration of sodium hydroxide is usually 0.4 to 10N, preferably 0.75 to 5N.

The pH of the bulk solution of the present invention is usually 5.5 to 7.0, preferably 6.0 to 6.6, and more preferably 6.1 to 6.5.

### (2) Lyophilized product

The lyophilized product is produced by filling a vial with the bulk solution obtained in (1), and then allowing water to sublimate. In general, lyophilization can be conducted by a primary drying step only, or by a primary drying step to remove free water and a secondary drying step to remove moisture and bound water that cannot be removed by the primary drying step. Here, when lyophilizing the present bulk solution, it is performed under optimized conditions, taking quality and production time into consideration. The present bulk solution can be dried by freezing at -50°C to -40°C, and then performing a primary drying step under high vacuum conditions (10 Pa or lower, preferably 5 Pa or lower) at a shelf temperature of 35°C to 55°C (preferably 40°C to 50°C). It is further preferable to control the appropriate temperature and vacuum, taking the quality uniformity of the production machine into consideration. For example, the present preparation is lyophilized under a vacuum of 5 Pa to 20 Pa and at a shelf temperature of -20°C to 25°C as the primary drying conditions, and then maintained under high vacuum conditions (10 Pa or lower, preferably 5 Pa or lower) and at a shelf temperature of 35°C to 55°C (preferably 40°C to 50°C) as the secondary drying conditions.

In the aforementioned procedures, there is no particular limitation with respect to the time for drying, which may vary depending on the size of the vial and the amount of the bulk solution filled in. Here, the time for drying is optimized, taking quality and production time into consideration, and is generally 5 hours to 1 week.

In the method of production of the lyophilized preparation of the present invention, either a primary drying step alone or a combination of a primary drying step and a secondary drying step may be performed; however, when quality uniformity is taken into consideration, the combination of a primary drying step and a secondary drying step is preferred.

### [Examples]

The present invention will be described in more detail with reference to Test Examples; however, the present invention shall not be limited to these.

### Test Example 1 Lyophilized product of formulations 1 to 3

Compound (I) as an active ingredient (11.5 g) and sodium chloride (none, 545 mg or 1.09 g) were weighed in a glass beaker. The beaker was ice-cooled, followed by addition of 30 mL of water for injection which was cooled to 5°C beforehand, and then the mixture was stirred using a stirrer to disperse compound (I) and to dissolve sodium chloride. 1N hydrochloric acid was added dropwise to the mixture while stirring under ice-cold conditions, to completely dissolve compound (I). Further, 1N hydrochloric acid was used to adjust the pH to 6.0. pH was measured using a pH meter (type: F-22, manufactured by HORIBA, Ltd.) (hereinafter the same shall apply). Water for injection was added to the solution under ice-cold conditions, so that the total amount became 100 g. The solution obtained was filtered using a 0.22 µm Millipore filter (Millidisk 10 cartridge filter, catalog No. MCGL10S03, manufactured by Nihon Millipore K.K.) to obtain a bulk solution.

Vials of 20 mL (size) were each filled with 5 g of the bulk solutions obtained, half stoppered with rubber stoppers, and loaded into a freeze-drier. Lyophilization was carried out under conditions of freezing temperature: -45°C, drying temperature: 45°C. After lyophilization, the headspaces of the vials were filled with nitrogen gas, followed with full stoppering, and then the vials were taken out of the freeze-drier. Lyophilized products of formulations 1 to 3 were prepared in accordance with the aforementioned lyophilization (formulation 1 is a comparative example).

The proportion of remaining active ingredient was determined for the case where the obtained lyophilized product was stored at 50°C for 8 weeks, and for the case where it was stored at 40°C for 2 months, according to the conditions given below. The proportion is presented as a ratio with respect to the amount of the active ingredient before storage. The results are shown in Table 2. In the table, "-" indicates that sodium chloride is not contained. As is obvious from Table 2, the preparations of the present invention which contain sodium chloride (formulations 2 and 3) show higher proportion remaining when compared with the preparation of the comparative example which does not contain sodium chloride (formulation 1).

### Method For Analyzing Proportion of Remaining Active Ingredient

Measured using HPLC under the following conditions.
HPLC system: SCL-10A (manufactured by Shimadzu Corporation)
Detector: SPD-10AC (UV spectrophotometer, manufactured by Shimadzu Corporation)
Column: Develosil RPAQUEOUS (4.6 mm I.D. x 150 mm length, manufactured by Nomura Chemical Co., Ltd.)
Column temperature: 60°C
Injection amount of sample: 10 µL
Flow rate of mobile phase: 1.0 mL/min
Mobile phase: 0.02 mol/L phosphate buffer (pH 7.0) / acetonitrile (19:1)
Measurement wavelength: 255 nm

**Table 2 Stability Test of Lyophilized Product of Formulations 1 to 3**

| Test Formulation | Formulation 1 | Formulation 2 | Formulation 3 |
|---|---|---|---|
| Compound (I) (mg) | 500 | 500 | 500 |
| NaCl (mg) | - | 27.25 | 54.5 |
| PH | 6.0 | 6.0 | 6.0 |
| Total amount (g) | 5 | 5 | 5 |

| Stability (proportion remaining, %) | | | |
|---|---|---|---|
| 50°C, 8 weeks | 91.9 | 93.3 | 96.5 |
| 40°C, 2 months | 98.6 | 99.5 | 101.4 |

### Test Example 2 Lyophilized product of formulations 4 to 8

Compound (I) as an active ingredient (60.05 g) and sodium chloride (2.83 g, 5.67 g, 8.50 g, 11.30 g or 14.20 g) were weighed in a glass beaker. The beaker was ice-cooled, followed by addition of 156 mL of water for injection which was cooled to 5°C beforehand, and then the mixture was stirred using a stirrer to disperse compound (I) and to dissolve sodium chloride. 1N hydrochloric acid was added dropwise to the mixture while stirring under ice-cold conditions, to completely dissolve compound (I). Further, 1N hydrochloric acid was used to adjust the pH to 6.0. Water for injection was added to the solution under ice-cold conditions, so that the total amount became 520 g. The solution obtained was filtered using a 0.22 µm Millipore filter to obtain a bulk solution.

Vials of 20 mL were each filled with 7.5 g of the bulk solutions obtained, lyophilized in a similar manner as Test Example 1, and thus lyophilized products of formulations 4 to 8 were obtained.

The proportion of remaining active ingredient and the total amount of related substances (for example, related substances with relative retention times of 0.5, 1.6, 1.8 and 1.9 with respect to the retention times of the main peak observed under the HPLC conditions below) were obtained for the case where the obtained lyophilized product was stored at 60°C for 8 weeks, according to the conditions given below. The results are presented as ratios with respect to the amount of the active ingredient before storage. The results are shown in Table 3. As is obvious from Table 3, the preparations of the present invention which contain sodium chloride (formulations 4 to 8) show high proportion of remaining active ingredient and low total amount of related substances even under severe conditions of 60°C for 8 weeks.

Method For Analyzing Proportion of Remaining Active Ingredient: the same as Test Example 1

Method For Analyzing Total Amount of Related Substances Measured using HPLC under the following conditions.
HPLC system: SCL-10A (manufactured by Shimadzu Corporation)
Detector: SPD-10AC (UV spectrophotometer, manufactured by Shimadzu Corporation)
Column: Develosil RPAQUEOUS (4.6 mm I.D. x 150 mm length, manufactured by Nomura Chemical Co., Ltd.)
Column temperature: 60°C
Injection amount of sample: 10 µL
Flow rate of mobile phase: 1.0 mL/min
Mobile phase A: 0.02 mol/L phosphate buffer (pH 7.0) / acetonitrile (19:1)
Mobile phase B: 0.02 mol/L phosphate buffer (pH 7.0) / acetonitrile (1:1)
Measurement wavelength: 210 nm

**Table 3 Stability Test of Lyophilized Product of Formulations 4 to 8**

| Test Formulation | Formulation 4 | Formulation 5 | Formulation 6 | Formulation 7 | Formulation 8 |
|---|---|---|---|---|---|
| Compound (I) (mg) | 750 | 750 | 750 | 750 | 750 |
| NaCl (mg) | 40.88 | 81.75 | 122.6 | 163.5 | 204.4 |
| PH | 6.0 | 6.0 | 6.0 | 6.0 | 6.0 |
| Total amount (g) | 7.5 | 7.5 | 7.5 | 7.5 | 7.5 |

| Stability (60°C, 8 weeks, %) | | | | | |
|---|---|---|---|---|---|
| Proportion remaining | 84.7 | 89.5 | 89.5 | 90.3 | 88.6 |
| Total amount of related substances | 14.8 | 9.2 | 8.5 | 8.0 | 10.0 |

### Test Example 3 Stability Test of Bulk Solution

Compound (I) as an active ingredient (5.96 g) and sodium chloride (0.55 g) were weighed in a glass beaker. The beaker was ice-cooled, followed by addition of 15 mL of water for injection which was cooled to 5°C beforehand, and then the mixture was stirred using a stirrer to disperse compound (I) and to dissolve sodium chloride. 1N hydrochloric acid was added dropwise to the mixture while stirring under ice-cold conditions, to completely dissolve compound (I). Further, 1N hydrochloric acid was used to adjust the pH to 6.0, 6.25, 6.5, 6.7 and 6.9. Water for injection was added to the solution under ice-cold conditions, so that the total amount became 50 g. The solution obtained was filtered using a 0.22 µm Millipore filter to obtain a bulk solution.

The proportion of remaining active ingredient and the total amount of related substances were obtained for the case where the obtained bulk solution was stored at 25°C for 2 days, according to the conditions given below. The results are presented as ratios with respect to the amount of the active ingredient before storage. The results are shown in Table 4. As is obvious from Table 4, the higher the pH, the higher the proportion of remaining active ingredient.

Method For Analyzing Proportion of Remaining Active Ingredient: the same as Test Examples 1 and 2

Method For Analyzing Total Amount of related Substances: the same as Test Example 2

**Table 4 Stability Test of Bulk Solution**

| Test Formulation | Formulation 9 | Formulation 10 | Formulation 11 | Formulation 12 | Formulation 13 |
|---|---|---|---|---|---|
| Compound (I) (mg) | 750 | 750 | 750 | 750 | 750 |
| NaCl (mg) | 81.75 | 81.75 | 81.75 | 81.75 | 81.75 |
| pH | 6.0 | 6.25 | 6.5 | 6.7 | 6.9 |
| Total amount (g) | 7.5 | 7.5 | 7.5 | 7.5 | 7.5 |

| Stability (25°C, 2 days, %) | | | | | |
|---|---|---|---|---|---|
| Proportion remaining | 80.9 | 84.9 | 86.7 | 88.3 | 88.7 |
| Total amount of related substances | 17.5 | 14.3 | 12.7 | 11.9 | 11.7 |

### [INDUSTRIAL APPLICABILITY]

The lyophilized preparation of the present invention is extremely useful in terms of practical use as a lyophilized preparation of a carbapenem compound, since it has superior storage stability.

## Claims

1. A lyophilized preparation comprising (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid or a pharmacologically acceptable salt thereof as a carbapenem compound, and sodium chloride.

2. The lyophilized preparation according to claim 1, wherein the amount of sodium chloride added is 0.1 to 2.5 equivalent (mol) with respect to the carbapenem compound.

3. The lyophilized preparation according to claim 1, wherein the amount of sodium chloride added is 0.5 to 2.0 equivalent (mol) with respect to the carbapenem compound.

4. The lyophilized preparation according to claim 1, wherein the amount of sodium chloride added is 0.7 to 1.8 equivalent (mol) with respect to the carbapenem compound.

5. A method of production of the lyophilized preparation according to any one of claims 1 to 4, comprising:
preparing a bulk solution by dissolving (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid or a pharmacologically acceptable salt thereof and sodium chloride in an aqueous solvent; and
lyophilizing the bulk solution.

6. A method of production of the lyophilized preparation according to any one of claims 1 to 4, comprising:
preparing a bulk solution by dissolving (1R, 5S, 6S)-6-[(1R)-1-hydroxyethyl]-1-methyl-2-[(2S, 4S)-2-[(3S)-3-(2-guanidinoacetylamino)pyrrolidin-1-ylcarbonyl]-1-methylpyrrolidin-4-ylthio]-1-carbapen-2-em-3-carboxylic acid, sodium chloride and other additive(s) if necessary in an aqueous solvent;
aseptically filtering the bulk solution;
filling the filtrate into a container; and
lyophilizing the filtrate.

7. A method of production of the lyophilized preparation according to any one of claims 1 to 4, comprising:
preparing a bulk solution by dissolving (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid, sodium chloride and other additive(s) if necessary in an aqueous solvent;
aseptically filtering the bulk solution;
lyophilizing the filtrate to obtain a lyophilized powder; and
filling the lyophilized powder into a container.

8. A method of production of the lyophilized preparation according to any one of claims 1 to 4, comprising:
preparing a bulk solution by dissolving (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid, sodium chloride and other additive(s) if necessary in an aqueous solvent;
aseptically filtering the bulk solution;
lyophilizing the filtrate to obtain a lyophilized powder; and
filling the lyophilized powder as a kit formulation.

9. The method of production according to any one of claims 5 to 8, wherein the bulk solution contains a pH regulator.

10. The method of production according to claim 9, wherein the pH regulator is selected from a hydrochloric acid solution of 0.4 to 20 N, and an aqueous sodium hydroxide solution of 0.4 to 10 N.

11. The method of production according to claim 9, wherein the pH regulator is selected from a hydrochloric acid solution of 0.75 to 5 N, and an aqueous sodium hydroxide solution of 0.75 to 5 N.

12. The method of production according to any one of claims 5 to 11, wherein the pH of the bulk solution is 5.5 to 7.0.

13. The method of production according to any one of claims 5 to 11, wherein the pH of the bulk solution is 6.0 to 6.6.

14. The method of production according to any one of claims 5 to 11, wherein the pH of the bulk solution is 6.1 to 6.5.

15. The method of production according to any one of claims 5 to 14, wherein the lyophilizing comprises only a primary drying step performed under high vacuum conditions of 10 Pa or lower, at a shelf temperature of 35°C to 55°C.

16. The method of production according to claim 15, wherein the high vacuum conditions are 5 Pa or lower.

17. The method of production according to claim 15 or 16, wherein the shelf temperature is 40°C to 50°C.

18. The method of production according to any one of claims 5 to 14, wherein the lyophilizing comprises a combination of a primary drying step under a vacuum of 5 Pa to 20 Pa and at a shelf temperature of -20°C to 25°C, and a secondary drying step under high vacuum conditions of 10 Pa or lower and at a shelf temperature of 35°C to 55°C.

19. The method of production according to claim 18, wherein the high vacuum conditions of the secondary drying step are 5 Pa or lower.

20. The method of production according to claim 18 or 19, wherein the shelf temperature of the secondary drying step is 40°C to 50°C.
